# EUROPÄISCHE PATENTSCHRIFT

(11) **EP 1 976 472 B1**
(45) Veröffentlichungstag und Bekanntmachung des Hinweises auf die Patenterteilung: **30.05.2012**
(21) Anmeldenummer: 07702928.8
(22) Anmeldetag: 22.01.2007
(51) Int. Cl.: A61F 13/00

(54) **VORGEFERTIGTE WUNDAUFLAGE MIT SUPERABSORBER**
PREFABRICATED WOUND DRESSING WITH SUPERABSORBER
PANSEMENT PRÉFABRIQUÉ CONTENANT UN SUPERABSORBANT

(30) Priorität: 25.01.2006 DE 202006001183 U; 15.05.2006 DE 202006007877 U
(43) Veröffentlichungstag der Anmeldung: 08.10.2008
(73) Patentinhaber: Riesinger, Birgit, 48346 Ostbevern (DE)
(72) Erfinder: Riesinger, Birgit, 48346 Ostbevern (DE)
(74) Vertreter: Storz, Ulrich
(86) Internationale Anmeldenummer: PCT/EP2007/000506
(87) Internationale Veröffentlichungsnummer: WO 2007/085396

(56) Entgegenhaltungen:
- EP-A1- 0 691 113
- WO-A-96/10972
- DE-A1-102004 002 990
- DE-U1-202004 017 052
- DE-U1-202004 018 245
- FR-A1- 2 846 635
- US-A- 3 871 376
- US-A- 3 872 862
- US-A- 5 480 646
- US-A1- 2004 127 826
- SHERMAN R A: "A new dressing design for use with maggot therapy" PLASTIC AND RECONSTRUCTIVE SURGERY, WILLIAMS AND WILKINS CO., BALTIMORE, MD, US, Bd. 100, 1997, Seiten 451-456, XP000905285 ISSN: 0032-1052

## Beschreibung

Die Erfindung betrifft eine Vorrichtung zur Wundbehandlung, aufweisend ein sich über den jeweiligen Wundbereich erstreckendes Wundabdeckungselement, das von wenigstens einem die Wundsekrete aufzunehmenden Absorptionskörper unterlegt ist, welcher Absorptionskörper wenigstens eine Lage eines mit Superabsorbentien durchsetzten Textilabschnittes ist, die mit einer feinperforierten, flüssigkeitsdurchlässigen Hülle umgeben ist, wobei unterhalb des umhüllten Absorptionskörpers - im am Körper des Patienten angelegten Zustand - wenigstens ein flüssigkeitsdurchlässiger, schleimhautfreundlicher Folienabschnitt angeordnet ist, mit dem sich ein Abstand zwischen der Wundoberfläche und dem umhüllten Absorptionskörper einhalten lässt.

Eine gattungsgemäße Vorrichtung ist aus der DE 20 2004 017 052 der Anmelderin bekannt. Der schleimhautfreundliche, hydrophobe Folienabschnitt schützt das Wundgewebe vor eventuellen schmerzhaften Berührungen mit dem Absorptionskörper bzw. mit der Hülle, insbesondere mit deren umlaufender Kante oder Naht. Er beugt der Verklebung des Absorptionskörpers mit dem Wundbett vor und verhindert die Mazeration. Der Einsatz der Vorrichtung erfordert eine Zuschneidung des Wundabdeckungselementes und des schleimhautfreundlichen Folienabschnittes aus den vorhandenen Materialien zwecks Anpassung an jeweilige Wundgröße. Zuerst wird der wundnahe Folienabschnitt entsprechend zugeschnitten und ins Wundbett lose gelegt. Erst danach werden der umhüllte Absorptionskörper und das unmittelbar vor der Behandlung entsprechend zugeschnittene wundabdeckungselement eingesetzt.

Ein ähnliches Verband-Aggregat zur gleichzeitigen Kühlung ist aus US 3,971,376 bekannt. Ein der Wunde zugewandter schleimhautverträglicher und flüssigkeitsdurchlässiger Folienabschnitt fehlt jedoch. Der beschriebene Absorptionskörper weist außerdem keinen speziellen mit z.B. Superabsorbentien durchsetzten Absorptionskörper auf.

Gleiches gilt für den in EP 0 691 113 A1 offenbarten wundverband, der ebenfalls keinen der Wunde zugewandten schleimhautverträglichen und flüssigkeitsdurchlässigen Folienabschnitt oder einen mit Superabsorbentien durchsetzten Absorptionskörper aufweist.

DE 20 2004 018 245 U1 offenbart zwar eine mit Unterdruck betriebene Drainagevorrichtung zur Wundbehandlung, die sowohl einen schleimhautfreundlichen Folienabschnitt als auch superabsorbierende Polymere aufweist, jedoch keinen mit speziellen Superabsorbentien durchsetzten Absorptionskörper.

Aufgabe der Erfindung ist, eine wundbehandlungsvorrichtung anzugeben, derer Einsatz weniger arbeitsintensiv ist.

Diese Aufgabe ist durch eine Wundbehandlungsvorrichtung der eingangs genannten Art gelöst, bei der der flüssigkeitsdurchlässige, wundnahe Folienabschnitt an seiner Peripherie mit dem Wundabdeckungselement umlaufend verbunden ist, wobei die Hülle des Absorptionskörpers innerhalb eines durch das Wundabdeckungselement und den Folienabschnitt gebildeten Beutels angeordnet ist.

Es wird ausdrücklich darauf hingewiesen, dass der besagte Folienabschnitt keine Flüssigkeite- und Dampfsperre, sondern ein die Wundexsudate in den Absorptionskörper abzuleitendes, biegsames Flachgebilde darstellt.

Durch die periphere Verbindung des Folienabschnittes mit dem Wundabdeckungselement ergibt sich eine einzigartige, beutelartige Wundauflage, in der der umhüllte Absorptionskörper lose und beweglich untergebracht ist.

Dabei kann die Hülle des Absorptionskörpers wenigstens punktweise mit dem Wundabdeckungselement verklebt sein, beispielsweise in ihrem Mittelbereich. Dies ist jedoch nicht unbedingt erforderlich, da die Bewegungsfreiheit der Hülle durch die genannte periphere Verbindung eingeschränkt ist.

Die Hülle des Absorptionskörpers besteht aus einem flüssigkeitsdurchlässigen Natur- oder Kunststoff, an dem die Wundsekrete nicht oder kaum haften. Dies ermöglicht den Transport von flüssigen Wundsekreten in das Absorptionsmaterial. Die Wundsekrete treten durch die Hülle hindurch und werden durch das mit Superabsorbentien angereicherte Absorptionsmaterial aufgesaugt. Daraus resultiert, dass die Vorrichtung gemäß Erfindung zwei Lagen von flüssigkeitsdurchlässigen Materialien aufweist, die - im am Körper des Patienten angelegten Zustand -unterhalb des in der Hülle befindlichen Absorptionskörpers liegen. Hierbei kann die Perforations- bzw. Maschengröße des wundnahen Folienabschnittes die der Hülle überschreiten.

Die periphere Verbindung kann auch einen umlaufenden Hüllenrand des Absorptionskörpers umfassen. In dem Fall entsteht ebenfalls eine beutelartige Wundauflage, bei der die Hülle des Absorptionskörpers mit ihrem Umfang am Folienabschnitt und am Wundabdeckungselement fixiert ist.

Das flüssigkeitsundurchlässige Wundabdeckungselement besteht vorzugsweise aus folienartigem, beispielsweise transparentem oder transluzentem Material. Das Wundabdeckungselement kann dampfdurchlässig sein.

Ferner ist es möglich, das flache, folienartige Wundabdeckungselement durch eine schalenförmige, ebenfalls folienartige und vorzugsweise transparente Abdeckung zu ersetzen, die mit einem Flachkragen zur Verbindung mit dem schleimhautfreundlichen Folienabschnitt und zum Ankleben an die Haut des Patienten versehen ist. Eine schalen- bzw. glockenförmige Abdeckung kann kostengünstig im Tiefziehverfahren hergestellt werden. Der Flachkragen kann an seiner dem Folienabschnitt zugewandten Unterseite eine Klebefläche aufweisen, die außerhalb des mit dem Flachkragen verklebten Folienabschnittes liegt und mit einer abziehbaren Schutzfolie versehen ist.

Das Wundabdeckungselement kann auf seiner Außenseite bedruckt sein.

Die periphere Verbindung kann durch mehrere entsprechend verteilte Klebe- oder Schweißpunkte oder durch wenigstens eine umlaufende Klebe- oder Schweißlinie bzw. durch wenigstens eine umlaufende Klebefläche gebildet sein.

Die Verklebung des Wundabdeckungselementes mit dem Folienabschnitt und optional mit der Hülle des Absorptionskörpers ermöglicht eine einfache Vorfertigung der Vorrichtung in unterschiedlichen Größen. Es können beispielsweise drei Typengrößen der Vorrichtung vorgesehen werden, die den typischen Flächengrößen: 5,5 cm x 5,5 cm; 7,5 cm x 7, 5 cm; 10,5 cm x 10,5 cm; 20,0 cm x 10,0 cm der Hülle des von der Anmelderin produzierten Absorptionskörpers entsprechen. Die Vorrichtung kann in einer sterilen Verpackung untergebracht sein und in dieser Form vertrieben werden.

Der Einsatz der Vorrichtung erfordert lediglich die Sterilisierung des Wundbetts und der gesunden Haut des Patienten um die wunde herum und Ankleben der ausgepackten Vorrichtung nach vorherigem Abziehen der Schutzfolie. Dadurch kann die Arbeitszeit des Fachpersonals.verkürzt und die Anbringung der Vorrichtung an der Wunde vereinfacht werden.

Vorteilhaft ist, dass die Vorrichtung keinen Sekundärverband und keine sonstigen Befestigungen erfordert.

Die Vorrichtung kann sowohl bei stationärer als auch bei ambulanter Wundbehandlung verwendet werden.

Die beschriebene Vorrichtung ist als Einweg-Wundauflage gedacht. Sollte jedoch der aufgequollene Absorptionskörper durch einen neuen, unbenutzten ersetzt werden während das Wundabdeckungselement noch an der Wunde sitzt, kann an diesem eine fensterartige, wieder verschließbare Öffnung vorgesehen sein, die das Auswechseln von Absorptionskörpern möglich macht. Die Öffnung kann beispielsweise durch einen einfachen Foliendeckel mit einer umlaufenden Release-Klebeschicht, wie bei den bekannten Peelpackungen der Fall ist, abgedeckt sein.

Der Wundheilungsprozess kann durch Unterdruck unterstützt werden. Zu diesem Zweck kann am Wundabdeckungselement wenigstens eine Öffnung eingebracht sein, an die ein den Unterdruck erzeugendes Gerät anschließbar ist. Das Gerät kann ein per Hand zusammendrückbarer Balg oder Ball sein, welcher schlauchlos oder über eine Leitung mit dem Wundabdeckungselement verbunden oder verbindbar ist. Es kann auch ein elektrisch oder mechanisch angetriebenes, handelsübliches Gerät angeschlossen sein.

Durch die andere am Wundabdeckungselement befindliche Öffnung kann eine Medikamentendosierung erfolgen. Die beiden Öffnungen können jeweils an einer am Wundabdeckungselement eingearbeiteten, vorzugsweise kegelförmigen Erhebung angeordnet sein, von denen wenigstens die eine Erhebung eine mit Medikamenten befüllbare Kammer bilden kann. Die Kammer kann ebenfalls im Tiefziehverfahren einstückig mit dem Wundabdeckungselement geformt sein.

Zur Medikamentendosierung kann ein vlies- oder schwammartiger, offenporiger Körper geeignet sein, mit welchem die Kammer befüllt ist. Das flüssige Medikament kann über eine Leitung und die vorhandene Öffnung dem vlies- oder schwammartigen Körper zugeführt werden.

Der Körper kann mit flüssigem Medikament bereits durchtränkt sein und in dieser Form in die Kammer platziert werden. In dem Fall ist keine Öffnung zur Medikamentendosierung und keine Aktivierung des Körpers durch Zugabe von Medikament erforderlich. Der mit Medikamenten durchsetzte Körper stellt also eine Patrone dar, die das enthaltene flüssige Medikament nach und nach abgibt. Hierbei scheint es möglich zu sein, eine per Hand zusammendrückbare folienartige Erhebung anzufertigen.

Der Absorptionskörper, dessen Hülle oder der schleimhautfreundliche Folienabschnitt können natürliche oder synthetisch gewonnene Hyaluronsäure, Honig oder dessen Derivate, Propolis und/oder pflanzliche Extrakte, wie Aloe vera, und/oder Absonderungen tierischer Provenienz, wie Madenspeichel, aufweisen.

Die Kammer kann durch ein perforiertes, ebenfalls folienartiges Bodenelement begrenzt sein, dessen Perforationen eine langsame, vorbestimmte Dosierung von Medikamenten ermöglichen.

Schließlich ist möglich, wenigstens den das Wundbett zu berührenden Folienabschnitt mit metallischen Nanopartikeln anzureichern. Die Nanopartikeln können beispielsweise in einer Materialschicht vorliegen, d. h. sie können mit der Materialschicht eine Matrix bilden. Die Materialschicht kann in flüssiger Phase auf den Folienabschnitt aufgetragen sein. Der Folienabschnitt kann beispielsweise in eine Lösung von Silbernitrat eingetaucht und danach ausgetrocknet werden. Auf jeden Fall soll der mit Nanopartikeln behandelte Folienabschnitt hydrophob und flüssigkeitsdurchlässig sein. Dies ist durchaus möglich, insbesondere dann, wenn der Folienabschnitt in Gewebeform alls so genanntes wunddistanzgitter vorliegt.

Ausführungsbeispiele der Erfindung sind anhand der Zeichnung näher erläutert. Die Figuren zeigen:
- Figuren 1a und 1b: eine erste Ausführungsform der Vorrichtung vor und nach dem Verbinden derer Teile miteinander, in einem schematischen Schnitt;
- Fig. 2: Detail einer peripheren Verbindung des Wundabdeckungselementes mit der Hülle des Absorptionskörpers und mit dem Folienabschnitt, in einem schematischen Schnitt;
- Fig. 3: eine andere Ausführungsform der Vorrichtung, enthaltend ein Anschlusselement zur Gasevakuierung, ebenso in einem schematischen Schnitt;
- Fig. 4: die Vorrichtung gemäß Fig. 1b in einer Explosionsdarstellung;
- Fig. 5: die Vorrichtung gemäß Fig. 1b in Draufsicht auf ihre Rückseite;
- Figuren 6a bis 6d: die Vorrichtung gemäß Fig. 3, angeklebt an die Haut des Patienten, in einem schematischen Schnitt;
- Fig. 7: einen Schnitt A-A gemäß Fig. 6d;
- Fig. 8: eine weitere Ausführungsform der Vorrichtung, mit zwei Hohlkammern, in einem schematischen Schnitt;
- Fig. 9: eine Vorrichtung mit schalenförmigem Wundabdeckungselement, ebenso in einem schematischen Schnitt, und
- Fig. 10: die Vorrichtung gemäß Fig. 1b mit Fenster.

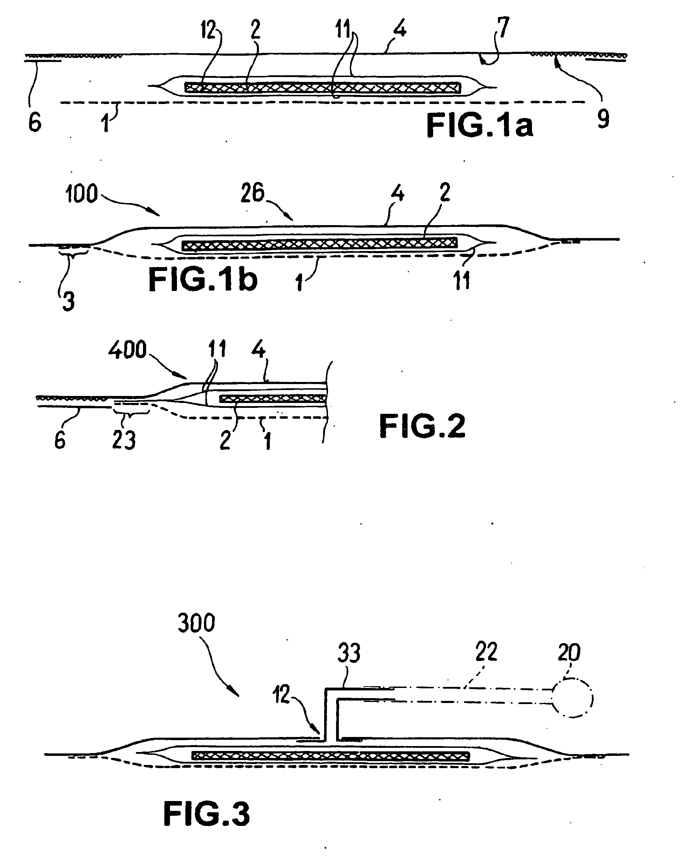

In den Figuren 1a, 1b, 4 und 5 ist eine Vorrichtung 100 zur Wundbehandlung dargestellt, bestehend aus einem folienartigen Wundabdeckungselement 4, einem schleimhautfreundlichen Folienabschnitt 1 und einem dazwischen liegenden Absorptionskörper 2.

Die Vorrichtung ist in Draufsicht auf ihre Flachseite etwa rechteckig und weist abgerundete Ecken 25 auf. An wenigstens einer Ecke des Wundabdeckungselementes 4 ist eine Ziehlasche 24 eingebracht. Außerdem ist eine sterile, luftdichte Verpackung (nicht dargestellt) vorgesehen.

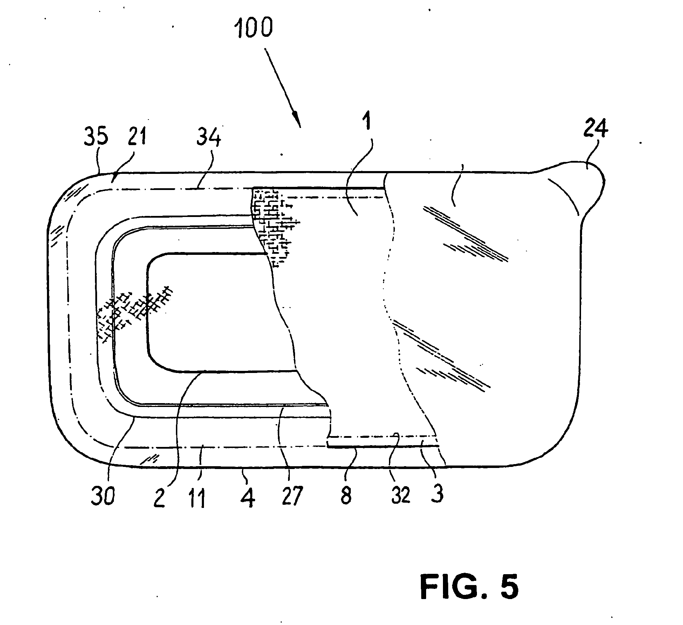

Das Wundabdeckungselement 4 ist an seiner Unterseite 7 mit einer peripher angeordneten Klebefläche 9 (vgl. Fig. 1a) zum Ankleben der Vorrichtung auf die Haut des Patienten und zugleich zum Verkleben mit dem Folienabschnitt 1 versehen. Verklebt ist der Folienabschnitt nur an seinem peripheren Rand 3, wobei die Verklebung sowohl durchgehend als auch punktweise erfolgt werden kann. Wie die Fig. 5 zeigt, ist der zu verklebende Rand 3 durch eine Außenkontur 8 des Folienabschnittes 1 und eine Strichpunktlinie 32 definiert.

Der äußerste Umfang der Klebefläche 9 ist mit einem umlaufenden, abziehbaren Schutzfolienstreifen 6 abgedeckt (vgl. Fig. 1a). Gemäß Fig. 5 ist der Schutzfolienstreifen durch eine abziehbare Folienabdeckung 31 ersetzt, welche ebenfalls peripher mit dem Wundabdeckungselement 4 lösbar verklebt ist. Eine umlaufende, lösbare Verklebung 21 der Folienabdeckung 31 mit dem Wundabdeckungselement 4 ist durch eine Außenkante 35 und eine Strichpunktlinie 34 (vgl. Fig. 5) definiert.

Anstelle einer festen kann eine lösbare Verklebung des Wundabdeckungselementes 4 mit dem Folienabschnitt 1, beispielsweise vermittels einer nicht dargestellten Release-Klebeschicht, zum Einsatz kommen.

Der Absorptionskörper 2 besteht aus einer Lage eines vliesartigen, textilen Materials, das Cellulosefasern umfaßt und mit Superabsorberteilchen (Super-Absorbing-Polymers, SAP), im vorliegenden Fall mit Natriumacrylat-Acrylsäure-Polymerisat durchsetzt ist. Außerdem ist der Absorptionskörper 2 mit nanokristallinen, silberhaltigen Substanzen angereichert, die mikrobizid wirken. Die Cellulosefasern wirken als Zwischenspeicher der spontan beaufschlagten Flüssigkeitsmengen und als eine Art Transportmittel, mit dem die Wundsekrete bis zum Superabsorber hin gelangen.

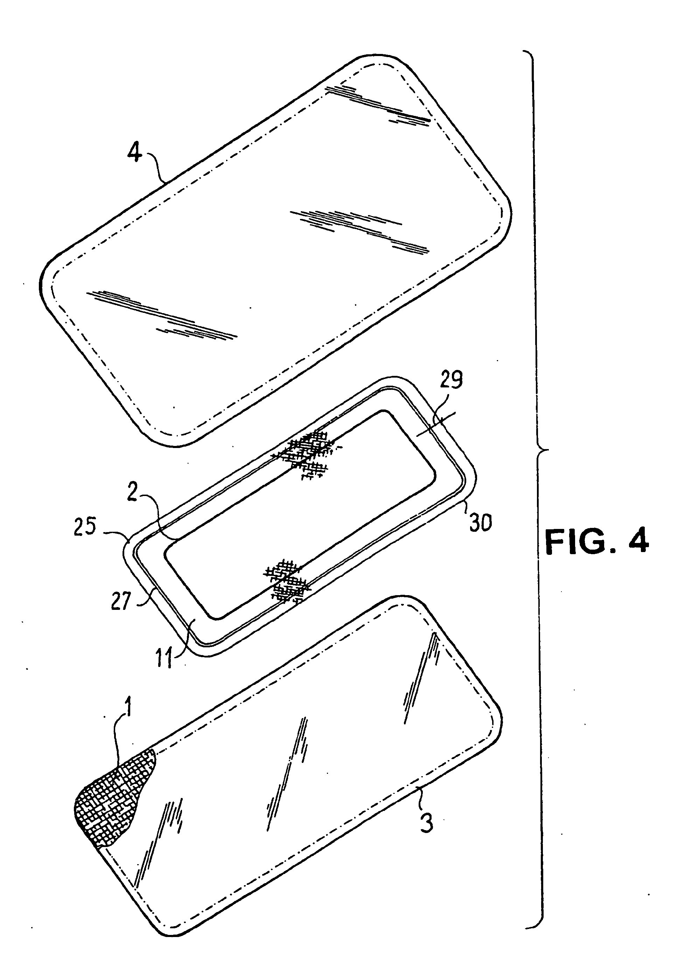

Die Lage des Absorptionskörpers 2 ist von einer flüssigkeitsdurchlässigen, ebenfalls textilen, mit einer umlaufenden Ultraschallnaht 27 verschweißten Hülle 11 umgeben. Wie insbesondere den Figuren 4 und 5 zu entnehmen ist, weist die Hülle 11 einen peripheren Überstand 29 an Hüllenmaterial auf, der zwischen der Ultraschallnaht 27 und einem äußersten Umfang 30 der Hülle 11 liegt. Die Aufgabe des Überstandes 29 ist, die Wunde vor der schmerzhaften Berührung mit der Naht zu verhindern.

Der wundnahe Folienabschnitt 1 ist aus einem flüssigkeitsdurchlässigen, hauchdünnen, schleimhautfreundlichen, gewebten Material aus Kunststofffasern angefertigt. Die Maschengröße des Folienabschnittes 1 liegt zwischen 0,5 mm und 1,0 mm. Der Folienabschnitt 1 trägt auch dem Schutz vor Berührung mit der Ultraschallnaht 27 bei. Durch die periphere Verklebung des Wundabdeckungselementes 4 mit dem Folienabschnitt 1 ergibt sich ein Beutel 26, in dem die Hülle 11 des Absorptionskörpers 2 lose untergebracht ist. Diese Konfiguration kann als "Beute1-Im-Beutel-System" bezeichnet werden.

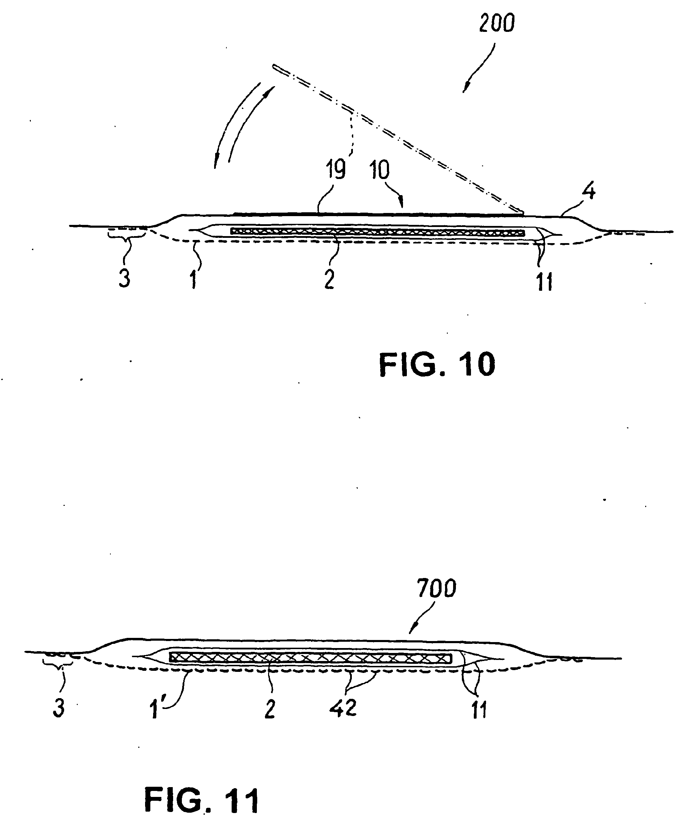

Eine weitere Entwicklung (Vorrichtung 200) der Ausführungsform gemäß Fig. 1b stellt die Fig. 10 dar. Gemäß Fig. 10 weist das Wundabdeckungselement 4 einen fensterartigen, schwenkbaren Foliendeckel 19 auf, welcher eine am Wundabdeckungselement 4 ausgeschnittene Öffnung 10 in Schließstellung luftdicht abdeckt. Diese Maßnahme erlaubt das Herausnehmen des aufgequollenen Absorptionskörpers aus dem äußeren Beutel, so dass der verbrauchte Absorptionskörper durch einen neuen ersetzt werden kann.

Die Fig. 3 zeigt eine Vorrichtung 300, die der Ausführung gemäß Fig. 1b sehr ähnlich ist. Am Wundabdeckungselement 4 ist eine mittige Öffnung 12 zur Aufnahme eines L-Stutzens 33 eingebracht, an den wiederum über eine Leitung 22 ein den Unterdruck erzeugendes, per Hand angetriebenes Gerät 20, wie zusammendrückbarer Balg, angeschlossen ist.

Fig. 2 zeigt eine Vorrichtung 400. Das Konzept der Vorrichtung 400 beruht auf Verklebung einer Peripherie 23 der Hülle 11 mit dem Folienabschnitt 1 und dem Wundabdeckungselement 4. Auf dieser Weise ist die flüssigkeitsdurchlässige Hülle 11 zwischen dem Wundabdeckungselement und dem wundnahen Folienabschnitt fixiert. Die vorrichtung 400 verfügt über die gleiche periphere Klebefläche zum Ankleben an die

Haut des Patienten und über den abziehbaren Schutzfolienstreifen 6.

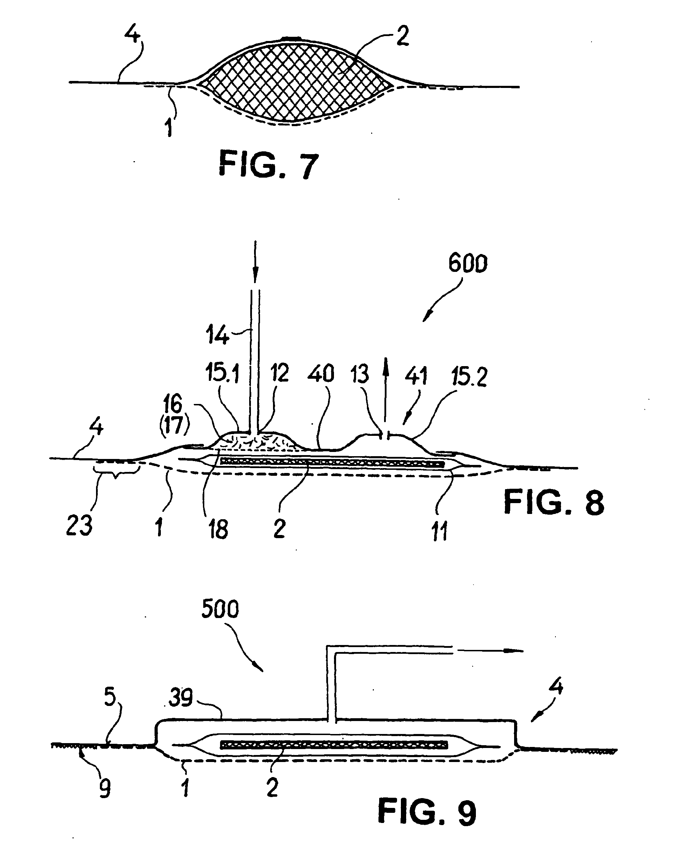

Eine der Ausführungsform gemäß Fig. 3 etwas abweichende Vorrichtung 500 ist in Fig. 9 dargestellt. Die Vorrichtung 500 unterscheidet sich von der in Fig. 3 gezeigten Vorrichtung 300 durch eine schalenförmige Ausbildung des Wundabdeckungselementes 4. Dementsprechend weist das Wundabdeckungselement ein im Wesentlichen planes Mittelteil 39 und einen herabgesetzten Flachkragen 5 auf, der wiederum direkt mit dem wundnahen Folienabschnitt 1 unter Belassung der freien peripheren Klebefläche 9 fest verklebt ist. Am planen Mitteteil kann ebenfalls ein schwenkbarer, fensterartiger Foliendeckel eingebracht sein.

Die Fig. 8 zeigt eine Vorrichtung 600, die wiederum eine Weiterentwicklung der Ausführungsform gemäß Fig. 2 darstellt. Am folienartigen, im Wesentlichen planen Wundabdeckungselement ist eine mittig angeordnete, breite Aussparung ausgeschnitten, welche durch ein Folienelement 40 abgedeckt ist. Das Folienelement 40 ist ebenfalls an seinem Umfang mit dem restlichen Wundabdeckungselement verklebt. Am Folienelement 40 sind zwei etwa sphärische Erhebungen 15.1, 15.2 vorgesehen, die jeweils eine Öffnung 12 bzw. 13 aufweisen. Die Erhebungen 15.1, 15.2 des Folienelementes 40 sind im Tiefziehverfahren geformt.

Die Erhebung 15.1 bildet eine mit Medikamenten befüllbare Kammer 16, die im vorliegenden Fall mit einem Körper 17 aus offenporigem Schaumstoff gefüllt ist. Das flüssige Medikament kann über eine Leitung 14 und die vorhandene Öffnung 12 dem schwammartigen Körper 17 zugeführt werden.

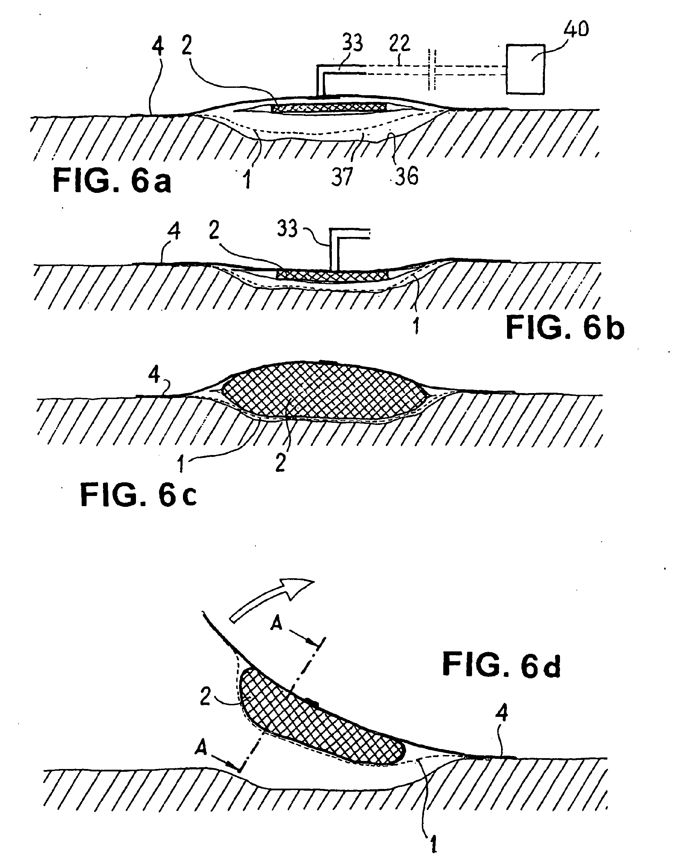

Über die zweite Öffnung 13 kann wiederum die Luft über eine Leitung 22, wie es in Fig. 6a gezeigt worden ist, abgeführt werden.

Optional kann die Kammer 16 durch ein perforiertes folienartiges Bodenelement 18 begrenzt sein.

Eine andere Ausführungsform (nicht dargestellt) sieht vor, in eine zweite, durch die zweite Erhebung gebildete Kammer ebenfalls einen schwammartigen, mit flüssigen Medikamenten durchtränkbaren Körper einzulegen.

Die Funktion wird nachfolgen anhand der Figuren 6a bis 6d beschrieben:

Eine tiefe Wunde 36 wird durch das Ankleben der Vorrichtung 300 (Einwegvorrichtung) gemäß Fig. 3 auf die Haut des Patienten vollständig bedeckt. Vorher wurde der in Fig. 1a gezeigte Schutzfolienstreifen 6 bzw. die in Fig. 5 gezeigte Folienabdeckung 31 entfernt, so dass die periphere Klebefläche 9 an der Unterseite des Wundabdeckungselementes 4 freigegeben wird. Durch das Ankleben der Vorrichtung auf die Haut ist ein Wundhohlraum 37 zwischen dem Wundabdeckungselementes 4 und der Wundoberfläche entstanden. An den L-Stutzen 33 wurde über die vorgenannte Leitung 22 ein elektrisch angetriebenes Vakuumgerät 38 angeschlossen. Da der Wundhohlraum 37 abgedichtet ist, können mit Hilfe des Vakuumgerätes 38 die im Wundhohlraum befindlichen Gase evakuiert werden. Den Zustand zeigt die Fig. 6b. Die flachen Elemente der Vorrichtung liegen nahezu auf der Wundoberfläche an. Der inzwischen abgelesene Unterdruck betrug etwa 100 ml Hg.

Die aus der Wunde heraustretenden Wundsekrete gelangen in den Absorptionskörper 2 und bewirken eine Kompression unterhalb des Wundabdeckungselementes 4. Nach dem Ansaugen von Wundsekreten nimmt das Volumen des Absorptionskörpers 2 stark zu (vgl. Fig. 6c). Da der Absorptionskörper 2 flächenmäßig etwa 40% kleiner als die Hülle 11 ist, nähert sich er einer Wurstform (vgl. Fig. 6d und 7) an.

Die verbrauchte Vorrichtung 300 wird jetzt durch das Anheben einer in Fig. 5 gezeigten Ziehlasche 24 vorsichtig aus dem Wundbereich entfernt. Nach Bedarf kann auf die Wunde eine neue Einwegvorrichtung aufgeklebt werden.

### Bezugszeichenliste:

- 1; 1': Folienabschnitt
- 2: Absorptionskörper
- 3: Rand (v. 1)
- 4: Wundabdeckungselement
- 5: Flachkragen
- 6: Schutzfolienstreifen
- 7: Unterseite
- 8: Außenkontur
- 9: Klebefläche
- 10: Öffnung
- 11: Hülle
- 12, 13: Öffnung
- 14: Leitung
- 15.1, 15.2: Erhebung
- 16: Kammer
- 17: Körper
- 18: Bodenelement
- 19: Foliendeckel
- 20: Gerät
- 21: Verklebung (lösbar)
- 22: Leitung
- 23: Peripherie (v. 11)
- 24: Ziehlasche
- 25: Ecke
- 26: Beutel
- 27: Ultraschallnaht (v. 11)
- 29: Überstand
- 30: Umfang
- 31: Folienabdeckung
- 32: Strichpunktlinie
- 33: L-Stutzen
- 34: Strichpunktlinie
- 35: Außenkante
- 36: Wunde
- 37: Wundhohlraum
- 38: Vakuumgerät
- 39: Mittelteil
- 40: Folienelement
- 41: Aussparung
- 42: Scheitel

- 100; 200; 300; 400: Vorrichtung
- 500; 600; 700: Vorrichtung

## Patentansprüche

1. Vorrichtung (100; 200; 300; 400; 500; 600; 700) zur Wundbehandlung, aufweisend ein sich über den jeweiligen Wundbereich erstreckendes Wundabdeckungselement (4), das von wenigstens einem die Wundsekrete aufzunehmenden Absorptionskörper (2) unterlegt ist, welcher Absorptionskörper (2) wenigstens eine Lage eines mit Superabsorbentien durchsetzten Textilabschnittes ist, die mit einer feinperforierten, flüssigkeitsdurchlässigen Hülle (11) umgeben ist, wobei zwischen dem Wundbett und dem umhüllten Absorptionskörpers (2) - im am Körper des Patienten angelegten Zustand - wenigstens ein flüssigkeitsdurchlässiger, schleimhautfreundlicher Folienabschnitt (1; 1') angeordnet ist, mit dem sich ein Abstand zwischen der Wundoberfläche und dem umhüllten Absorptionskörper einhalten lässt,
**dadurch gekennzeichnet, dass**
- der Folienabschnitt (1; 1') an seinem Rand (3) mit dem Wundabdeckungselement (4) umlaufend verbunden ist,
- die Hülle (11) des Absorptionskörpers (2) innerhalb eines durch das Wundabdeckungselement (4) und den Folienabschnitt (1; 1') gebildeten Beutels (26) angeordnet ist,
- und der Textilabschnitt mit Superabsorbentien aus einem Polymerisat aus Natriumacrylat und Acrylsäure durchsetzt ist.

2. Vorrichtung nach Anspruch 1, **dadurch gekennzeichnet, dass** der Folienabschnitt (1; 1') mit dem Wundabdeckungselement (4) lösbar verbunden ist.

3. Vorrichtung nach Anspruch 1, **dadurch gekennzeichnet, dass** der Folienabschnitt (1; 1') an seinem Rand (3) durchgehend oder stellenweise mit dem Wundabdeckungselement (4) verklebt oder verschweißt ist.

4. Vorrichtung nach Anspruch 1, **dadurch gekennzeichnet, dass** die Hülle (11) des Absorptionskörpers an seiner Peripherie (23) stellenweise mit dem Folienabschnitt (1; 1') und dem Wundabdeckungselement (4) verbunden ist.

5. Vorrichtung nach Anspruch 1, **dadurch gekennzeichnet, dass** das Wundabdeckungselement (4) planar oder schalenförmig ist.

6. Vorrichtung nach Anspruch 5, **dadurch gekennzeichnet, dass** das schalenförmige Wundabdeckungselement (4) in einen Flachkragen (5) übergeht, mit dem der Folienabschnitt (1; 1') verbunden ist.

7. Vorrichtung nach einem der vorhergehenden Ansprüche, **dadurch gekennzeichnet, dass** das Wundabdeckungselement (4) an seiner dem Folienabschnitt (1) zugewandten Unterseite (7) eine Klebefläche (9) aufweist, die außerhalb des Folienabschnittes (1; 1') liegt und mit einem abziehbaren Schutzfolienstreifen (6) oder einer Folienabdeckung (31) versehen ist.

8. Vorrichtung nach einem der vorhergehenden Ansprüche, **dadurch gekennzeichnet, dass** am Wundabdeckungselement (4) wenigstens eine Öffnung (12; 13; 10) eingearbeitet ist.

9. Vorrichtung nach Anspruch 8, **dadurch gekennzeichnet, dass** die Öffnung (10) wieder verschließbar und durch einen fensterartigen Foliendeckel (19) luftdicht abgedeckt ist.

10. Vorrichtung nach Anspruch 8, **dadurch gekennzeichnet, dass** an die Öffnung (12) ein den Unterdruck erzeugendes Gerät (20; 21) angeschlossen oder anschließbar ist.

11. Vorrichtung nach Anspruch 10, **dadurch gekennzeichnet, dass** an die Öffnung (12; 13) eine Leitung (14) zur Medikamentendosierung anschließbar ist.

12. Vorrichtung nach einem der vorhergehenden Ansprüche, **dadurch gekennzeichnet, dass** wenigstens eine der Öffnungen (12; 13) an einer am Wundabdeckungselement (4) befindlichen Erhebung (15.1, 15.2) angeordnet ist.

13. Vorrichtung nach Anspruch 12, **dadurch gekennzeichnet, dass** die Erhebung (15.1) eine mit Medikamenten befüllbare Kammer (16) bildet.

## Claims

1. A device for wound treatment (100; 200; 300; 400; 500; 600; 700), comprising a wound covering element (4) that extends over the respective wound zone and is supported by at least one absorption body (2) for receiving the wound secretions, whereas the absorption body (2) consisting of at least one layer of a textile section that is impregnated with superabsorbers, said layer being surrounded with a finely perforated liquid-permeable sleeve (11), wherein at least one liquid-permeable film section (1; 1'), acceptable to the mucous, is arranged between the wound bed and the enveloped absorption body (2) - when applied to the body of the patient - so that a distance between the wound surface and the enveloped absorption body can be maintained
**characterized in that**
the film section (1; 1') is connected at its edge (3) to the wound covering element (4) over its entire periphery,
the sleeve (11) of the absorption body (2) is movably arranged inside a bag (26) formed by the wound covering element (4) and the film section (1; 1'), and
the textile section that is impregnated with superabsorbers comprises a polymerizate comprising sodium acrylate and acrylic acid.

2. The device according to claim 1, **characterized in that** the film section (1; 1') is removably attached to the wound covering element (4).

3. The device according to claim 1, **characterized in that** the film section (1; 1') at its edge (3) is over its entire periphery or only partially glued or bonded to the wound covering element (4).

4. The device according to claim 1, **characterized in that** the sleeve (11) of the absorbent body is at its periphery (23) partially connected to the film section (1; 1') and the wound covering element (4).

5. The device according to claim 1, **characterized in that** the wound covering element (4) is planar or dish-shaped.

6. The device according to claim 5, **characterized in that** the dish-shaped wound covering element (4) fits into a flat collar (5), which is connected to the film section (1; 1').

7. The device according to any of the preceding claims, **characterized in that** the back side (7) of the wound covering element (4) facing into the direction of the film section (1) comprises outside of the film section area (1; 1') a glued surface (9), which is equipped with a detachable protective tape (6) or a foil cover (31).

8. The device according to any of the preceding claims, **characterized in that** the wound covering element (4) comprises at least one opening (12; 13; 10).

9. The device according to claim 8, **characterized in that** the opening (10) is resealable and air-tightly sealed by a window-like film cover (19).

10. The device according to claim 8, **characterized in that** the opening (12) is connected or is suitable for connection to the negative pressure generating apparatus (20; 21).

11. The device according to claim 10, **characterized in that** the opening (12; 13) is suitable for connection to a tube (14) for medication purposes.

12. The device according to any of the preceding claims, **characterized in that** at least one of the openings (12; 13) is located at a protrusion (15.1; 15.2) of the wound covering element (4).

13. The device according to claim 12, **characterized in that** the protrusion (15.1) forms a medicine-fillable compartment (16).

## Revendications

1. Dispositif (100 ; 200 ; 300 ; 400 ; 500 ; 600 ; 700) pour le traitement d'une plaie, comportant un élément (4) couvrant la plaie, lequel s'étend sur la zone de plaie respective et en dessous duquel se trouve au moins un corps absorbant (2) recevant les sécrétions de la plaie, lequel corps absorbant (2) est constitué d'au moins une couche d'un morceau de textile contenant un superabsorbant qui est entourée d'une enveloppe (11) finement perforée et perméable aux liquides, au moins un morceau de film (1 ; 1') qui est perméable aux liquides et toléré par les muqueuses et avec lequel peut être respectée une distance entre la surface de la plaie et le corps absorbant enveloppé placé entre la plaie et le corps absorbant enveloppé (2) - lorsque celui-ci est appliqué sur le corps du patient -,
**caractérisé**
- **en ce que** le morceau de film (1 ; 1') est assemblé par son bord (3) sur toute sa périphérie à l'élément (4) couvrant la plaie,
- **en ce que** l'enveloppe (11) du corps absorbant (2) est placée mobile à l'intérieur d'un sachet (26) formé par l'élément (4) couvrant la plaie et par le morceau de film (1 ; 1'),
- et **en ce que** le morceau de textile contient un superabsorbant constitué d'un polymérisat et d'acide acrylique.

2. Dispositif selon la revendication 1, **caractérisé en ce que** le morceau de film (1 ; 1') est assemblé de manière amovible à l'élément (4) couvrant la plaie.

3. Dispositif selon la revendication 1, **caractérisé en ce que** le morceau de film (1 ; 1') est collé ou soudé par son bord (3), de manière continue ou par points, à l'élément (4) couvrant la plaie.

4. Dispositif selon la revendication 1, **caractérisé en ce que** l'enveloppe (11) du corps absorbant est assemblée par sa périphérie (23), par points, au morceau de film (1 ; 1') et à l'élément (4) couvrant la plaie.

5. Dispositif selon la revendication 1, **caractérisé en ce que** l'élément (4) couvrant la plaie est planaire ou en forme de cuvette.

6. Dispositif selon la revendication 5, **caractérisé en ce que** l'élément (4) couvrant la plaie et en forme de cuvette se transforme en un col plat (5) auquel le morceau de film (1 ; 1') est assemblé.

7. Dispositif selon l'une des revendications précédentes, **caractérisé en ce que**, sur sa face inférieure (7) proche du morceau de film (1), l'élément (4) couvrant la plaie comporte une surface adhésive (9) qui se situe en dehors du morceau de film (1 ; 1') et qui est munie d'une bande de film protecteur (6) pouvant être retirée ou d'un film de couverture (31).

8. Dispositif selon l'une des revendications précédentes, **caractérisé en ce qu'**au moins une ouverture (12 ; 13 ; 10) est pratiquée dans l'élément (4) couvrant la plaie.

9. Dispositif selon la revendication 8, **caractérisé en ce que** l'ouverture (10) peut être refermée et être couverte, de manière étanche à l'air, par un film formant couvercle (19) à la manière d'une fenêtre.

10. Dispositif selon la revendication 8, **caractérisé en ce qu'**un appareil (20 ; 21) produisant une dépression est ou peut être raccordé à l'ouverture (12).

11. Dispositif selon la revendication 10, **caractérisé en ce qu'**un conduit (14) destiné au dosage de médicaments peut être raccordé à l'ouverture (12 ; 13).

12. Dispositif selon l'une des revendications précédentes, **caractérisé en ce qu'**au moins l'une des ouvertures (12 ; 13) est placée sur une bosse (15.1, 15.2) située sur l'élément (4) couvrant la plaie.

13. Dispositif selon la revendication 12, **caractérisé en ce que** la bosse (15.1) forme une chambre (16) pouvant être remplie de médicaments.
